# EUROPEAN PATENT SPECIFICATION

(11) **EP 3 982 749 B1**
(45) Date of publication and mention of the grant of the patent: **15.07.2026**
(21) Application number: 19812771.4
(22) Date of filing: 29.11.2019
(51) Int. Cl.: A23K 10/18, A23K 50/30, A23K 50/60, A61K 36/064

(54) **METHODS FOR GROWING THE POPULATION OF MICROORGANISMS IN A GUT OF MONOGASTRIC ANIMALS**
VERFAHREN FÜR DAS WACHSTUM EINER POPULATION VON MIKROORGANISMEN IM DARM VON MONOGASTRISCHEN TIEREN
PROCÉDÉS PERMETTANT D'ACCROÎTRE LA POPULATION DE MICRO-ORGANISMES DANS LE TUBE DIGESTIF D'ANIMAUX MONOGASTRIQUES

(30) Priority: 14.06.2019 EP 19305767
(43) Date of publication of application: 20.04.2022
(60) Divisional application: 26182945.1
(73) Proprietor: Danstar Ferment AG, 6300 Zug (CH)
(72) Inventor: APPER, Emmanuelle, 31702 Blagnac (FR); CASTEX, Mathieu, 31702 Blagnac (FR); ACHARD, Caroline, 31702 Blagnac (FR)
(74) Representative: J A Kemp LLP
(86) International application number: PCT/EP2019/083172
(87) International publication number: WO 2020/249246

(56) References cited:
- US-B1- 6 841 149
- RIMA HATOUM ET AL: "Antimicrobial and Probiotic Properties of Yeasts: From Fundamental to Novel Applications", FRONTIERS IN MICROBIOLOGY, vol. 3, 1 January 2012 (2012-01-01), XP055642706, DOI: 10.3389/fmicb.2012.00421
- ALESSANDRO AGAZZI: "The Beneficial Role of Probiotics in Monogastric Animal Nutrition and Health", JOURNAL OF DAIRY, VETERINARY & ANIMAL RESEARCH, vol. 2, no. 4, 27 June 2015 (2015-06-27), XP055642709, DOI: 10.15406/jdvar.2015.02.00041
- VOHRA ASHIMA ET AL: "Probiotic yeasts in livestock sector", ANIMAL FEED SCIENCE AND TECHNOLOGY, ELSEVIER, AMSTERDAM, NL, vol. 219, 31 May 2016 (2016-05-31), pages 31 - 47, XP029679277, ISSN: 0377-8401, DOI: 10.1016/J.ANIFEEDSCI.2016.05.019

## Description

### FIELD

The present invention relates to non-therapeutic methods and uses for growing the population of microorganisms in the gut of offspring of a monogastric animal.

### BACKGROUND

It is known that healthy, disease-free animals grow faster or are more able to convert their feed efficiently into body tissue than sick or immune-challenged animals. Obviously, faster growth or a more efficient conversion of feed into desirable body tissue in an animal is both economically and ecologically important, especially in animals raised for food. For this, and other reasons, it is desirable to prevent animals from contacting diseases.

One approach to keeping animals healthy is to give the animals antibiotics. However, that approach is not desirable for animals raised for food because there can be antibiotic residues in the food. Furthermore, using antibiotics increases the risk to select antibioresistant bacteria, which is a human health concern of crucial importance.

In another approach, live yeast supplementation to ruminants has been shown to enhancing fiber digestion and improve the growth thereof. A number of in vivo experiments have demonstrated in ruminants the effect of live yeast in enhancing fiber digestion (Wohlt et al., 1988; Guedes et al., 2008; Marden et al., 2008), and have concluded that one of the main mechanisms by which this is achieved is by increasing the growth and activities of fibrolytic bacteria community, including *Fibrobacteres succinogenes* (Chaucheyras-Durand and Fonty, 2002; Mosoniet al., 2007; Wallace and Newbold, 2007). *Fibrobacteres* is a bacterial phylum first described in ruminants. Only the genus *Fibrobacter* has been described for this phylum, and this genus presently contains only formally cultured and described species, *Fibrobacter intestinalis* and *Fibrobacter succinogenes. Fibrobacteres* is known to possess a unique array of hemicellulose-degrading enzymes and is an efficient and prolific degrader of cellulose as its sole energy source (Suen et al., 2011).

The knowledge about *Fibrobacteres* in monogastrics, and more particurlarly in swine is however less advanced. Culture-dependent approaches using selective medium and culture-independent techniques targeting 16S rDNA identified a potential core population inhabiting the swine gut population, including the genera *Fibrobacter. Fibrobacter intestinalis* and *Fibrobacter succinogenes* have been both identified in the gut of swine. Very little is known about interactions between probiotics and *Fibrobacteres* in monogastric animals. The only published result is a study about supplementation of post-weaned piglets for 28 days post-weaning with a probiotic bacteria leads to a decrease of *Fibrobacteres* population (Li et al., 2016). Contrary to what it is known and observed in ruminants, the effects of yeast on the activity and the growth of fibrolytic community, and more precisely of *Fibrobacteres,* in monogastric animals are not documented.

Another low studied phylum is the phylum of *Actinobacteria,* a phylum considered as minor in terms of relative abundance, but for which importance of the functionality is more and more claimed. The family of *Bifidobacteriaceae,* an important fibrolytic family is part of the *Actinobacteria.* Similarly, the family of *Coriobacteraceae* belongs to the phylum of *Actinobacteria.* Several bacteria that are part of this family are involved in the biliary acids metabolism, an interesting target to promote feed efficiency and growth performance of animals.

Fiber degradation is important for monogastric animal, such as, for example swine, as among other benefits it produces short-chain fatty acids (SCFA), an energy source for the colonocytes.

In the same manner, conversion of primary biliary acids into secondary biliary acids to improve growth performance (Ipharaguerre et al., 2018) is also growing in importance. Modulating gut microbiota to promote balance between primary and secondary biliary acids is thus interesting to optimise feed utilization and growth performance of monogastric animals.

The phyla *Fibrobacteres* and *Actinobacteria* are minor bacterial phyla in terms of relative abundance, however, they can have a really important functional role due to the fact that they exert fibrolytic effects and that the phylum of *Actinobacteria* contain bacteria able to convert bile acids (for example *Collinsella sp.* and *Olsenella sp*.). In the gut, *Coriobacteriaceae* carry out functions of importance such as the conversion of bile salts and steroids as well as the activation of dietary polyphenols (Clavel et al., *2014). Coriobacteriaceae* is a family within the order *Coriobacteriales* (phylum *Actinobacteria*)*.*

There is therefore a need for maintaining or growing the population of microorganisms responsible of fiber digestion, conversion of bile acids, or both in a gut of monogastric animals and/or a litter thereof to enhance fiber digestion of the monogastric animals. There is also an increased need for enhancing average daily gain of monogastric animals.

US 6,841,149 B1 describes a probiotic mixture intended for monogastric animals to control intestinal flora populations.

### SUMMARY OF THE INVENTION

The scope of the invention is defined by the claims. Any aspects and examples of the present disclosure which do not fall under the scope of the appended claims do not form part of the invention and are merely provided for illustrative purposes.

The present invention provides a non-therapeutic use of a composition for maintaining or growing the population of microorganisms in the gut of offspring of a monogastric animal, wherein the monogastric animal is a sow, wherein the population of microorganisms is from the *Fibrobacteres* phylum, wherein the composition comprises at least one biologically pure culture of *Saccharomyces cerevisiae var boulardii* strain deposited under accession number I-1079 at the CNCM in an amount effective for maintaining or growing the population of microorganisms in the gut of the offspring, and a suitable carrier, and wherein the composition does not comprise any *Enterococcus* strain, and wherein the use comprises feeding the composition to the sow during gestation and/or lactation.

In certain embodiments of the non-therapeutic use of the invention, the population of microorganisms in the gut of the offspring of the sow fed with the composition is enhanced by at least 2, 3, 4, 5, 10, 15, 20, 25, 30, 35, 40, 45, 50, 55, 60, 65, 70, 75, 80, 85, 90, 95 or 100% when compared to the microorganism population of offspring of a sow not fed with the composition.

The present invention further provides a non-therapeutic method of maintaining or growing a population of microorganisms in the gut of offspring of a monogastric animal, the method comprising feeding to the monogastric animal during gestation and/or lactation an effective amount of a composition, wherein the monogastric animal is a sow, wherein the population of microorganisms is from the *Fibrobacteres* phylum, wherein the composition comprises at least one biologically pure culture of *Saccharomyces cerevisiae var boulardii* strain deposited under accession number I-1079 at the CNCM and a suitable carrier, and wherein the composition does not comprise any *Enterococcus* strain.

In certain embodiments of the non-therapeutic method of the invention, (a) the growth of the population in the gut of the offspring of the sow is enhanced by at least 2, 3, 4, 5, 10, 15, 20, 25, 30, 35, 40, 45, 50, 55, 60, 65, 70, 75, 80, 85, 90, 95 or 100% when compared to the microorganism population of offspring of a sow not fed with the composition; (b) the method comprises admixing the composition with the basal diet of the animal; (c) the step of feeding to the sow an effective amount of the composition is done on a daily basis for a period of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 days; and/or (d) the composition fed to the sow further comprises at least one antibiotic, Zinc Oxide or a mixture thereof. The antibiotic may be Amoxicillin, Doxycycline, Lyncomycin, colistin, Tiamulin, or any combinations thereof.

In certain embodiments of the non-therapeutic use or the non-therapeutic method of the invention, the microorganisms are from the *Fibrobacteres* genus. The microorganisms may be a *Fibrobacter intestinalis* strain.

In certain embodiments of the non-therapeutic use or the non-therapeutic method of the invention, (a) the suitable carrier is feed; (b) the at least one biologically pure culture of *Saccharomyces cerevisiae var boulardii* strain is in an amount of at least 1x10⁹ CFU per kilogram of the composition; and/or (c) the composition is in the form of a gelatin capsule, a pressed tablet, a gel cap, an animal feed, or a liquid beverage.

In certain embodiments of the non-therapeutic use or the non-therapeutic method of the invention, the composition further comprises at least one additional microorganism strain.

In certain embodiments of the non-therapeutic use or the non-therapeutic method of the invention, the composition does not comprise any bacterial strain and/or any additional yeast strain, or does not comprise any additional microorganism strain.

The present disclosure also describes a composition comprising at least one biologically pure culture of *Saccharomyces cerevisiae* strain in amount effective for enhancing an average daily gain of monogastric animals, and a suitable carrier.

The present disclosure also describes a composition comprising at least one biologically pure culture of *Saccharomyces cerevisiae* strain in amount effective for maintaining or growing a population of microorganisms in a gut of monogastric animals and/or a litter thereof, and a suitable carrier. The population of microorganisms may be responsible for fiber digestion, for conversion of primary biliary acids into secondary biliary acids or both.

The present disclosure further describes a feed or food additive comprising the composition as defined in the embodiments of the present disclosure.

The present disclosure also describes a use of the composition as defined in the embodiments of the present disclosure, for enhancing an average daily gain of monogastric animals.

The present disclosure also describes a method of maintaining or growing the population of microorganisms in a gut of monogastric animals and/or a litter thereof, the method comprising feeding to the monogastric animals an effective amount of the composition of the present disclosure.

The present disclosure also describes a method for enhancing an average daily gain of monogastric animals, the method comprising feeding to the monogastric animals an effective amount of the composition as defined in the embodiments of the present disclosure.

### DESCRIPTION OF THE FIGURES

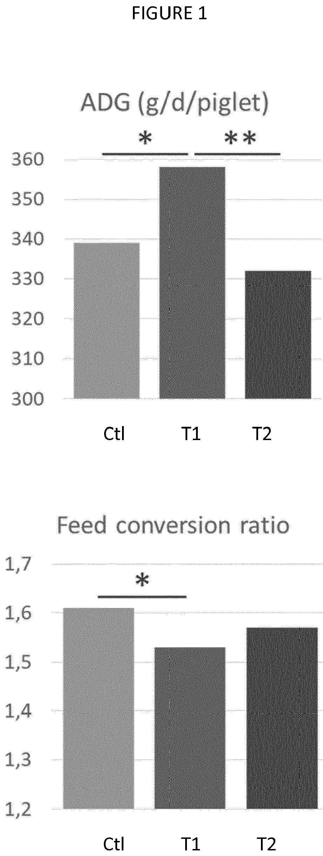

Figure 1 shows the Average Daily Gain (ADG) and feed conversion ratio obtained in piglets fed antibiotics alone (Control (Ctl)); *Saccharomyces cerevisiae var. boulardii* CNCM I-1079 combined with antibiotics (T1), and *Saccharomyces cerevisiae var. boulardii* CNCM I-1079 without antibiotics (T2) during the entire trial.

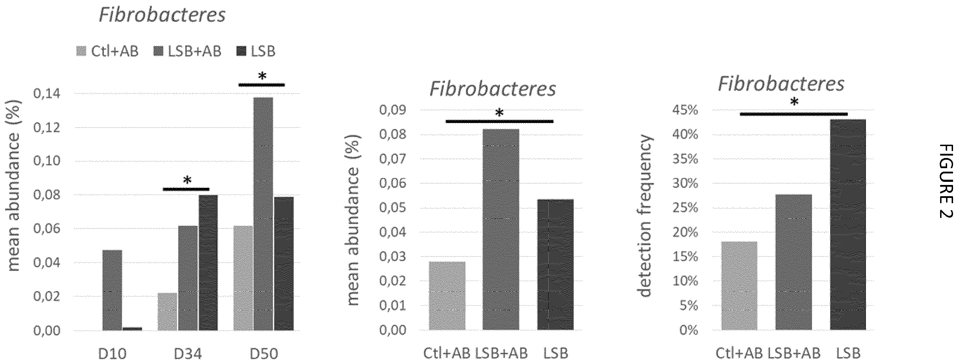

Figure 2 shows the mean abundance and detection frequency of *Fibrobacteres* phylum in piglets fed with antibiotics alone (Control (Ctl+AB)); *Saccharomyces cerevisiae var. boulardii* CNCM I-1079 combined with antibiotics (LSB+AB), and *Saccharomyces cerevisiae var. boulardii* CNCM I-1079 without antibiotics (LSB) at different time (Day (D) 10, 34 and 50) and on average. The first plot (on the left) shows the results per time points (Day 10, 34 and 50). The second plot (in the middle) shows the same result expressed on average per treatment during the whole experiment and the last figure (on the right) shows the average detection frequency per treatment.

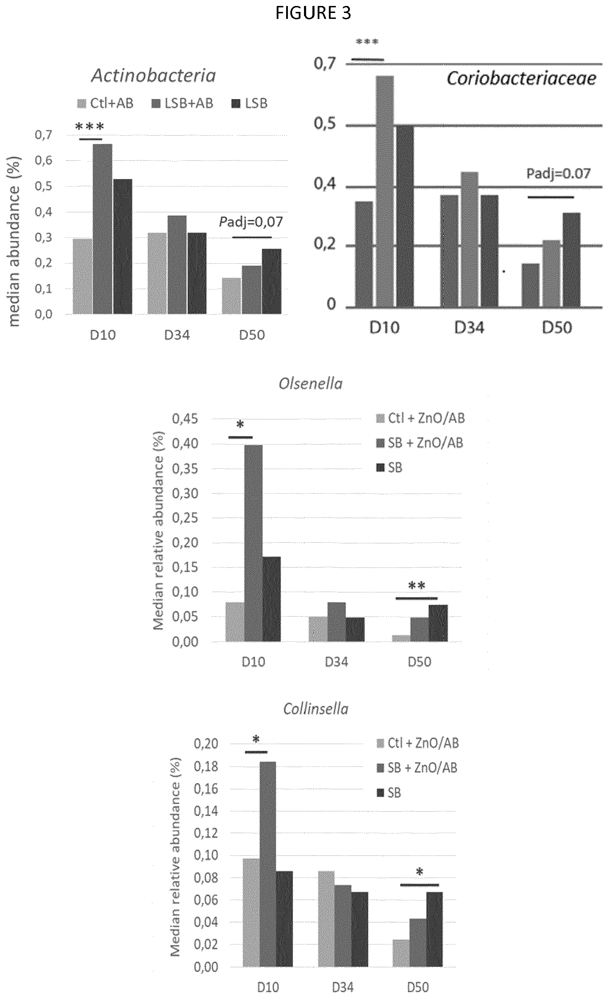

Figure 3 shows the median abundance of *Actinobacteria* phylum, *Coriobacteriaceae* family, and *Collinsella sp. and Olsenella sp.* in piglets fed with antibiotics alone (Ctl+AB ); *Saccharomyces cerevisiae var. boulardii* CNCM I-1079 + Antibiotitics (LSB+AB ), and *Saccharomyces cerevisiae var. boulardii* CNCM I-1079 alone (LSB).

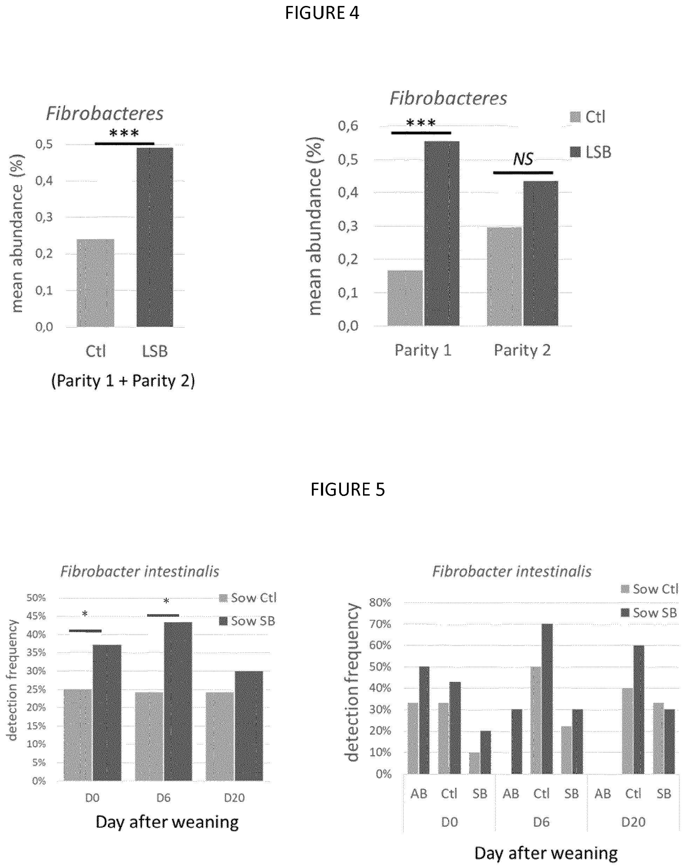

Figure 4 shows the mean abundance of *Fibrobacteres* in sows fed with Control diet (Ctl) or with *Saccharomyces cerevisiae var. boulardii* CNCM I-1079 (LSB), on average (left-hand panel) and per parity (right hand panel).
Figure 5 - On the left: Detection frequency of *Fibrobacter intestinalis* in piglets from mothers fed either with Control diet (Ctl) or with *Saccharomyces cerevisiae var. boulardii* CNCM I-1079 (LSB). On the right: effect of the treatment received by the sow (Ctl or LSB) and by the piglets (AB: Positive control, with antibiotic; Ctl: Negative control, without antibiotic and SB: *Saccharomyces cerevisiae var. boulardii* CNCM I-1079 diet) on the detection frequency of *F. intestinalis* per time points (day 0, 6, and 20). The antibiotic given to the piglets results in no detection of *F. intestinalis* in piglet feces, except when the sow was fed with *Saccharomyces cerevisiae var. boulardii* CNCM I-1079. When piglets received the negative control (Ctl), the supplementation of the sows with *Saccharomyces cerevisiae var. boulardii* CNCM I-1079 results in an increase of the detection frequency of *F. intestinalis* in piglet feces.

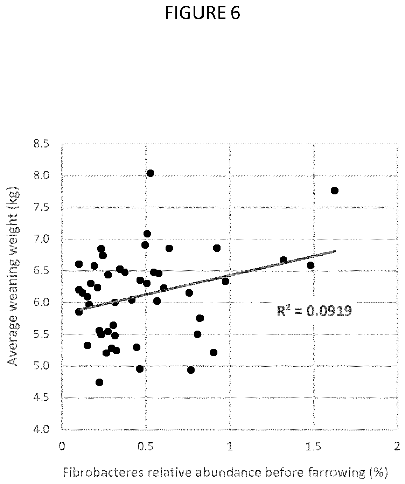

Figure 6 shows positive correlation of Fibrobacteres relative abundance in sows before farrowing (%) and average weaning weight of piglets (kg).

### DETAILED DESCRIPTION

Most nutrients from the feed are chemically digested and absorbed by the small intestine. This is the case, in general, for protein, lipids, and digestible carbohydrates. However, a big portion of the non-digestible carbohydrates will reach the hindgut where they will be partially consumed by the local microbial communities, the microbiota. Short Chain Fatty Acids (SCFAs) are formed as a product of this microbial fermentation, and can be absorbed locally and used as a source of energy for the host. The fiber fraction of the feed is very heterogeneous and may include:
Soluble components, which are easily fermented such as, for example, fructans, gums and/or pectins;
Partially degradable structural components such as cellulose and/or hemicellulose;
Cell wall protecting substances, which are practically indigestible, like chitin and/or lignin.

The inclusion of fiber in swine diets stimulates the speed of the digestive transit in relation with its content in Neutral Detergent Fiber (NDF) and benefits animal welfare, reducing constipation incidence, stereotypic behaviors and stress (Gerrits and Verstegen, 2006).

The proportion of fermentable fiber is positively associated with the content of soluble fiber and negatively associated with the level of lignin. It is related with changes in the intestinal environment (pH, ammonia concentration, production of SCFAs). Both types of fiber, soluble low lignified (e.g., sugar beet pulp) and insoluble lignified (e.g., oats bran) affect swine intestinal health through different mechanisms: a) production of SCFA from hindgut fermentation, and b) improvement of intestinal motility and functionality (Nutritional requirements for swine, FEDNA, 2013)

Sows are well adapted to digest fiber. They are equipped with a more voluminous large intestine than piglets or fattening pigs. The digesta remains in the large intestine for 70-85% of the total digestion time, allowing it to be in contact with the hindgut microbiota. This particularity confers sows a much higher cellulolytic activity than young pigs for example. In fact, many of the bacteria able to digest fiber that are located in the rumen of a cow can also be identified in the colon of sows. It is important to note that cellulolytic bacteria need an anaerobic environment to proliferate and be metabolically active. However, this is not always the case due to the huge vascularization irrigating the intestinal mucosa that brings in oxygen and can have a negative impact on the microbial profile. The increase in fibrolytic bacteria population in the sows could be of interest also for the piglets, as it is well-demonstrated that the microbiota of the sows strongly influences the early colonization in the piglet.

Biliary acids emerge as a promising target for developing efficacious alternatives to the use of antibiotic as growth promoter. Indeed, it has been demonstrated that the use of antibiotics and zinc oxide at doses commonly used for stimulating growth or preventing post-weaning enteritis in pigs converge in promoting microbial production of bile acids (BA) in the intestine. This leads to tissue - specific modifications in the proportion of BA, thereby amplifying BA signaling in intestine, liver, and white adipose tissue. Activation of BA-regulated pathways ultimately reinforces the intestinal protection against bacterial infection and pathological secretion of fluids and electrolytes, attenuates inflammation in colon, alters protein and lipid metabolism in liver. Conceivably, these alterations could spare nutrients for growth and improve the metabolic efficiency of treated monogastric animals. Thus, promoting bacterial population able to produce these bile acids is of interest for promoting growth of animals.

The present disclosure follows from the unexpected finding that feeding monogastric animals with a *Saccharomyces cerevisiae* strain, or a composition comprising thereof impacts on feed efficiency of monogastric animals and on an average daily weight gain of monogastric animals by enhancing fiber digestibility of monogastric animals by influencing lower gut fermentation; and also by stimulating a bacterial population related to bile acids metabolism.

Without being bound to a particular theory, it is believed that part of the mode of action of *Saccharomyces cerevisiae* in the gut of monogastric animals has to do with the fast consumption of oxygen carried out by the yeast in both caecum and colon. This creates better anaerobic conditions where the anaerobic bacteria, comprising cellulolytic bacteria and bacteria involved in the bile acids metabolism can proliferate. As a result, more energy in the form of SCFA is released from the same diet and in a shorter time; while the change in the bile acids metabolism leads to better growth efficiency.

The present disclosure describes a composition comprising at least one biologically pure culture of *Saccharomyces cerevisiae* strain in amount effective for enhancing average daily gain of monogastric animals, and a suitable carrier.

The present disclosure further describes a composition comprising at least one biologically pure culture of *Saccharomyces cerevisiae* strain in amount effective for maintaining or growing a population of microorganisms in a gut of monogastric animals and/or a litter thereof, and a suitable carrier. In an embodiment, the population of microorganisms is either responsible for fiber digestion, of conversion of primary biliary acids into secondary biliary acids or both.

The at least one biologically pure culture of *Saccharomyces cerevisiae* strain may be a probiotic strain. The at least one biologically pure culture of *Saccharomyces cerevisiae* strain is a *Saccharomyces cerevisiae var. boulardii* strain. According to the present invention, the at least one biologically pure culture of *Saccharomyces cerevisiae* strain may be a *Saccharomyces cerevisiae var. boulardii* strain deposited under accession number 1-1079 at the CNCM (i.e. at the COLLECTION NATIONALE DE CULTURES DE MICROORGANISMES, Institut Pasteur, 25-28 rue du Docteur Roux, 75724 Paris Cedex 15, FRANCE).

The expression "in amount effective" when used herein will be understood to refer to an amount of at least one biologically pure culture of *Saccharomyces cerevisiae* strain which is at least sufficient to maintain or grow the population of microorganisms in a gut of monogastric animals and/or a litter thereof. The expression also refer to an amount of at least one biologically pure culture of *Saccharomyces cerevisiae* strain which is at least sufficient to enhance an average daily gain of monogastric animals.

The amount of at least one biologically pure culture of *Saccharomyces cerevisiae* strain which is at least sufficient to maintain or grow the population of microorganisms in a gut of monogastric animals and/or a litter thereof; or to enhance an average daily gain of offspring from monogastric animals may be an amount of at least 1x10⁷ CFU per kilogram of the composition, at least 1x10⁸ per kilogram of the composition or 1x10⁹ CFU per kilogram of the composition. The at least one biologically pure culture of *Saccharomyces cerevisiae* strain may be in an amount ranging from 1x10⁷ CFU to 1x10¹² CFU per kilogram of the composition. The at least one biologically pure culture of *Saccharomyces cerevisiae* strain may be in an amount ranging from 1x10⁸ CFU to 1x10¹² CFU per kilogram of the composition. The at least one biologically pure culture of *Saccharomyces cerevisiae* strain may be in an amount ranging from 1x10⁹ CFU to 1x10¹² CFU per kilogram of the composition.

In an embodiment of the methods and uses of the present invention, the suitable carrier is feed, more specifically any orally ingestible animal feed suitable for monogastric animals. The skilled person in the art will appreciate that feed may vary from one monogastric animals to another. The feed including but not limited to a soup, pellets or a meal-based diet for swine and poultry, or kibbles, wet food and treats for dogs and cats. At least one biologically pure culture of *Saccharomyces cerevisiae var boulardii* CNCM I-1079 can be admixed with the basal diet of the sow. Alternatively, the at least one biologically pure culture of *Saccharomyces cerevisiae var boulardii* CNCM I-1079 can be top-dressed over feed. The at least one biologically pure culture of *Saccharomyces cerevisiae var boulardii* CNCM I-1079 can also be top-dressed over the basal diet of the monogastric animals.

The monogastric animals described herein may be swine, dogs, cats, horses, rabbits or poultry. The swine described herein may include sows, growing and fattening pigs and piglets. According to the present invention, the monogastric animal is a sow. The poultry described herein may be hens and chicks. The monogastric animals may be offsprings thereof.

In one embodiment, the compositions used in the methods and uses of the present invention can further comprise at least one additional microorganism strain. The additional microorganism strain including but not limited to *Bacillus subtilis, B. amyloliquefaciens, B. licheniformis, Enterococcus faecium, Pediococcus acidilacti, Lactococcus lactis, Lactobacillus acidophilus, L. casei, L. plantarum, L. rhamnosus* or a mixture thereof.

The compositions comprising at least one biologically pure culture of *Saccharomyces cerevisiae var boulardii* CNCM I-1079 used in the methods and uses of the present invention do not comprise any *Enterococcus* strain. The compositions may not comprise any bacterial strain and/or may not comprise any additional yeast strain. The compositions may not comprise any additional microorganism strain. The composition may consist or consist essentially of one or more biologically pure culture of *Saccharomyces cerevisiae* strain, and a suitable carrier.

The compositions in accordance with the present description can be in any suitable form to be served to the monogastric animals. The skilled person in the art would know what form is preferable for each monogastric animal species. For the sake of exemplifying the various form available, the compositions in accordance with the present disclosure can be in the form of a gelatin capsules, a pressed tablets, a gel caps, an animal feed or supplements, animal food or liquid beverages.

The expression "population of microorganisms responsible of fiber digestion" when used herein will be understood to refer generally to microorganisms that are able to process complex plant polysaccharides thanks to their capacity to synthesize cellulolytic and hemicellulolytic enzymes (often referred to as *Fibrolytic* bacteria). Polysaccharides are present in plant cellular cell walls in a compact fiber form where they are mainly composed of cellulose and hemicellulose.

The expression "population of microorganisms responsible of conversion of primary biliary acids into secondary biliary acids" when used herein will be understood to refer generally to microorganisms that are able to metabolize primary biliary acids. Biliary acids are produced from cholesterol and other acid steroids by the liver of mammals.

The population of microorganisms responsible of fiber digestion is from the *Fibrobacteres* phylum. The population of microorganisms responsible of fiber digestion may be from the Fibrobacter genus.

The population of microorganisms responsible of fiber digestion may be a *Fibrobacter intestinalis,* a *Fibrobacter succinogenes,* or both.

In either cases, the population of microorganisms also allows for enhancing an average daily gain of monogastric animals.

The present disclosure further describes a feed or a food additive comprising a composition as defined previously.

The present disclosure further provides for a use of the composition described hereinabove to enhance fiber digestibility of monogastric animals. The fiber digestibility of monogastric animals fed with the composition as defined above enhanced fiber digestibility of monogastric animals by at least 1, 2, 3, 4, 5, 10, 15, 20, 25, 30, 35, 40, 45, 50, 55, 60, 65, 70, 75, 80, 85, 90, 95 or 100% when compared to the fiber digestibility of a monogastric animal not fed with the composition.

The composition described herein may be used for maintaining or growing the population of microorganisms in a gut of monogastric animals and/or a litter thereof. The population of microorganisms in a gut of monogastric animals fed with the composition may be enhanced by at least 10, 25, 30, 35, 40, 45, 50, 55, 60, 70, 80, 85, 90, 100, 105, 110, 115, 120, 125, 130, 135, 140, 145, 145, 150, 155, 160, 165, 170, 175, 180, 185, 190, 200, 205, 210, 215, 220, 225, 230, 235, 240, 245, 250, 255, 260, 265, 270, 275, 280, 285, 290, 295 or 300% when compared to the microorganism population in a monogastric animal not fed with the composition. The population of microorganisms may be responsible of fiber digestion, of conversion of primary biliary acids into secondary biliary acid or both.

The composition described herein may be used for enhancing an average daily gain of monogastric animals. The average daily gain of monogastric animals fed with the composition may be enhanced of at least 1, 2, 3, 4, 5, 10, 15, 20, 25, 30, 35, 40, 45, 50, 55, 60, 65, 70, 75, 80, 85, 90, 95 or 100% when compared to the average daily gain of monogastric animals not fed with the composition.

The present disclosure further provides for a method of maintaining or growing the population of microorganisms in a gut of monogastric animals and/or a litter thereof, the method comprises feeding to the monogastric animals an effective amount of the composition as defined in the embodiments above. The population of microorganisms allows for or is responsible of fiber digestion, of conversion of primary biliary acids into secondary biliary acids or both. The growth of the population of microorganisms in the gut of monogastric animals may be enhanced by at least 10, 15, 20, 25, 30, 35, 40, 45, 50, 55, 60, 70, 80, 85, 90, 100, 105, 110, 115, 120, 125, 130, 135, 140, 145, 145, 150, 155, 160, 165, 170, 175, 180, 185, 190, 200, 205, 210, 215, 220, 225, 230, 235, 240, 245, 250, 255, 260, 265, 270, 275, 280, 285, 290, 295 or 300% when compared to the microorganism population in a monogastric animal not fed with the composition. In another embodiment of the method, the composition can be admixed with the basal diet of the animal.

The amount of at least one biologically pure culture of *Saccharomyces cerevisiae* strain may be in an amount of at least 1x10⁷ CFU per kilogram of the composition, at least 1x10⁸ per kilogram of the composition or 1x10⁹ CFU per kilogram of the composition. The at least one biologically pure culture of *Saccharomyces cerevisiae* strain may be in an amount ranging from 1x10⁷ CFU to 1x10¹² CFU per kilogram of the composition. The at least one biologically pure culture of *Saccharomyces cerevisiae* strain may be in an amount ranging from 1x10⁸ CFU to 1x10¹² CFU per kilogram of the composition. The at least one biologically pure culture of *Saccharomyces cerevisiae* strain may be in an amount ranging from 1x10⁹ CFU to 1x10¹² CFU per kilogram of the composition.

In an embodiment of the method, the step of feeding to the monogastric animal an effective amount of the composition is done on a daily basis. In yet a further embodiment of the method, the step of feeding to the monogastric animals an effective amount of the composition is done on a daily basis for a period of at least 10, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 days.

In an embodiment of the method, the step of feeding to the monogastric animals an effective amount of the composition is done at least 5 days before whelping. It was found that feeding monogastric animal with the composition in accordance with the present description was also beneficial for the gut of offspring.

The present invention provides a non-therapeutic method of maintaining or growing a population of microorganisms in the gut of offspring of a monogastric animal, the method comprising feeding to the monogastric animal during gestation and/or lactation an effective amount of a composition, wherein the monogastric animal is a sow, wherein the population of microorganisms is from the *Fibrobacteres* phylum, wherein the composition comprises at least one biologically pure culture of *Saccharomyces cerevisiae var boulardii* strain deposited under accession number I-1079 at the CNCM and a suitable carrier, and wherein the composition does not comprise any *Enterococcus* strain.

In an embodiment, the growth of the population of microorganisms is enhanced by at least 2, 3, 4, 5, 10, 15, 20, 25, 30, 35, 40, 45, 50, 55, 60, 70, 80, 85, 90, or 100% when compared to the microorganism population in the offspring of a sow not fed with the composition.

In an alternate embodiment of the method described in the embodiment above, the step of feeding the composition to the sows can be done in combination with at least one antibiotic and/or Zinc Oxide. The antibiotics are those usually used by in agriculture for growing monogastric animals. The antibiotic used can be, for example, but not limited to is Amoxicillin, Doxycycline, Lyncomycin, colistin, Tiamulin, or any combinations thereof. Other suitable antibiotics can also be used in combination with the composition in accordance with the present disclosure in the method described above.

In an embodiment, the population of microorganisms enhances average daily gain of the offspring of the sow.

The average daily gain of offspring from monogastric animals fed with an effective amount of composition in accordance with the present disclosure may be increase within a at least 21 days period compared to the average daily gain of offspring from monogastric animals not fed with an effective amount of the composition. The average daily gain of offspring from monogastric animals fed with the composition may be enhanced of at least 1, 2, 3, 4, 5, 10, 15, 20, 25, 30, 35, 40, 45, 50, 55, 60, 65, 70, 75, 80, 85, 90, 95 or 100% when compared to the average daily gain of offspring from monogastric animals not fed with the composition.

In any of the uses and methods described above, the composition comprising at least one biologically pure culture of *Saccharomyces cerevisiae* strain may not comprise any *Enterococcus* strain. The composition may not comprise any bacterial strain and/or may not comprise any additional yeast strain. The composition may not comprise any additional microorganism strain. The methods and uses may not comprise administration of any *Enterococcus* strain. The methods and uses may not comprise administration of any additional bacterial strain and/or any additional yeast strain. The methods and uses may not comprise administration of any additional microorganism strain,

The uses described in accordance with the invention are non-therapeutic. The methods described above in accordance with the invention are non-therapeutic.

The monogastric animals of the present disclosure may be swine, dogs, cats, horses, rabbits, poultry or offspring thereof. The swine may be sows, growing and fattening pigs, and piglets. The monogastric animals can be in gestation, in lactation, weaning or growing. According to the present invention, the monogastric animal is a sow and the use and method comprises feeding the composition to the sow during gestation and/or lactation.

### EXAMPLES:

### Example 1 - Post-weaning piglets supplemented with Saccharomyces cerevisiae var. boulardii CNCM I-1079

### Materials and methods

Fecal samples were collected during a 50 day trial conducted on 288 piglets after weaning. Piglets were weaned at 21 day of age and followed a 3-phase feeding program. Basal diet was supplemented with antibiotics and ZnO in the first two phases (D0-D11 and D12-D33) and only with antibiotics in the third phase (D34-D50). Piglets were allocated to three groups: basal diet, basal diet supplemented with 2x 10⁹ CFU/kg *Saccharomyces cerevisiae var. boulardii* CNCM I-1079, diet without medication after D11 and with 2x 10⁹ CFU/kg *Saccharomyces cerevisiae var. boulardii* CNCM I-1079. Fecal samples were collected from 24 piglets per treatment group at D10, D34 and D50. Zootechnical performances were also measured: body weight (BW) was individually registered at the beginning and at the end of the trial and at each diet change. The feed intake (FI) was measured per pen at each change of diet.

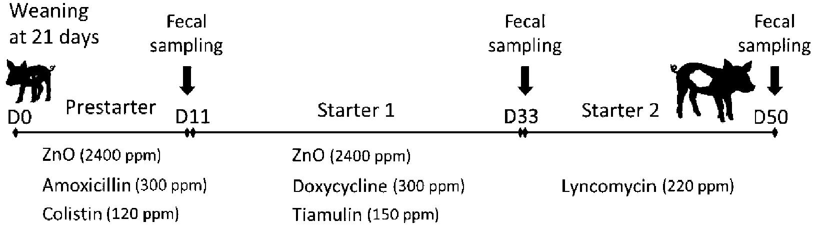

| **Treatment** | **Period (post-weaning)** | | |
|---|---|---|---|
| | **P1: 0-11** | **P2: 12-33** (3 weeks) | **P3: 34-50** (2,5 weeks) |
| **CTL+AB** | AB + ZnO | AB + ZnO | AB |
| **LSB+AB** | AB + ZnO + **LSB** | AB + ZnO + **LSB** | AB + **LSB** |
| **LSB** | AB + ZnO + **LSB** | **LSB** | **LSB** |

### Microbial DNA extraction and 16S rRNA gene sequencing

Fecal samples collected were processed using a DNA extraction kit from Qiagen. Microbial DNA was extracted from 40-60 mg of feces using ZR-96 Soil Microbe DNA Kit^{™} (Zymo Research, Freiburg, Germany) according to the manufacturer's instruction. A 15 min bead beating step at 30 Hz was applied using a Retsch MM400 Mixer Mill. The V3 and V4 hypervariable regions of the 16S rRNA gene were amplified using the following primers:
**V3-V4 region**
V3V4-343F 5'- CTTTCCCTACACGACGCTCTTCCGATCTACGG**R**AGGCAGCAG -3'
V3V4-783R 5'- GGAGTTCAGACGTGTGCTCTTCCGATCTTACCAGGGTATCTAATCCT -3'

High-throughput sequencing was performed on a MiSeq sequencer using the Reagent Kit v3, according to the manufacturer's instruction (Illumina Inc., San Diego, CA).

### Bioinformatics analysis

Bioinformatics analyses were performed using GenoToul bioinformatics facility (Toulouse, France). Generated paired-end 250 bp sequences were assembled using Flash software (10bp minimum overlap, 10% maximum mismatch). Assembled sequences were processed using FROGS pipeline (Escudié et al., 2018). Briefly, sequences were clustered in Operational Taxonomic Units (OTUs) using SWARM algorithm (Mahé et al., 2014). Chimeric sequences were then detected by samples using UCHIME algorithm and removed from all samples (Edgar et al., 2011). A rarefaction step was applied to each sequencing dataset to avoid bias due to differences in sequencing depth. A filtering step was applied to remove all singletons (i.e. OTU represented by only one read). The generated OTU count table was normalized by total sum scaling. Taxonomic annotation of the OTUs was performed using the SILVA SSU database and BLAST+ and RDP algorithms. BLAST hits with identity and coverage alignments higher than 99% were kept for annotation. Otherwise, species were annotated as unknown and RDP classifier results were used for higher rank. Bootstrap thresholds were set to 0,9 and 0,8 respectively for annotation at the genus rank and higher ranks. Phylum, family and genus relative abundance tables were generated.

### Statistical analysis

*Zootechnical performances.* Average daily gain (ADG), feed intake (FI), and feed conversion ratio (FCR) were performed by a MIXED procedure of SAS (The SAS Stat. v.9.3) for repeated measurements on pen basis. The statistical model accounted for the main effects of treatment, sex, and time, also considering the interaction between treatment and time, treatment and sex, and treatment x time x sex. The experimental unit was the pen. Significance level was fixed for A,B P≤0.01 and a,b P≤0.05, while 0.05<P≤0.1 was considered as a trend.

*Microbiota analysis.* Microbiota statistical analyses were carried out using R software and RStudio software. A Kruskal-Wallis Rank Sum Test followed by Pairwise Test for Multiple Comparisons of Mean Rank Sums (Conover-Test, PMCMR R package) were used to analyze compositional data. Differential analyses were performed only on taxa represented by more than 0,005% of the total sequences. The Benjamini-Hochberg procedure was used to adjust generated P values. Contingency data were analyzed using a Fisher's exact test.

### Results

*Zootechnical performances.* Overall, there were significant differences (P<0.01) found in average daily gain (ADG), piglets fed T1 (yeast + antibiotic) being the ones growing faster than piglets fed Ctl (Ctl + antibiotic) and T2 (yeast alone) (Figure 1). There was an effect found in Feed Conversion Ratio (FCR), with piglets fed T1 converting the feed into body weight gain better than piglets fed Ctl (Figure 1). No significant differences were found for the feed intake, all piglets ate the same quantity of feed whatever the treatments.

*Microbiota analysis.* The relative abundance of the phyla *Fibrobacteres* increased with time (P<0,001) whatever the treatment was, probably due to the increase of fiber quantity reaching the colon.

The supplementation with yeast resulted in an increase of *Fibrobacteres* mean abundance (expressed as %) and in *Fibrobacteres* detection frequency (in %, P<0,05; Figure 2). This means that when piglets were supplemented with *Saccharomyces cerevisiae var. boulardii* CNCM I-1079, the abundance of Fibrobacteres in their colon was greater and that the presence of *Fibrobacteres* was more frequently detected in the colon of these piglets than in the control piglets.

The relative abundance of the phylum *Actinobacteria* and of the family of *Coriobacteriaceae* was also found to be increased in feces from piglets fed with *Saccharomyces cerevisiae var. boulardii* CNCM I-1079 on day 10 and on day 50 (Figure 3). The family *Coriobacteriaceae* was the most dominant family, accounting for more than 80%, among the Actinobacteria phylum. Among the *Coriobacteriaceae,* two genera, *Olsenella* and *Collinsella* increased on D10 and on D50 in piglets fed with *Saccharomyces cerevisiae var. boulardii* CNCM I-1079 (Figure 3).

### Example 2 - Sows supplemented with Saccharomyces cerevisiae var. boulardii CNCM 1-1079 during gestation and lactation

### Materials and methods

### Experimental design

Fecal samples were collected during a trial conducted on sows (from 4 weeks before farrowing until weaning) and associated post-weaning piglets. Sows were fed a diet supplemented or not with 10⁹ CFU/kg *Saccharomyces cerevisiae var. boulardii* CNCM I-1079 **(Ctl** or **LSB** diet respectively). Piglets were weaned at 21 day of age and followed a 2-phase feeding program. Piglets born from control or *Saccharomyces cerevisiae var. boulardii* CNCM I-1079-treated sows were subsequently allocated to three groups: basal diet (**Ctl**), basal diet supplemented with 2500ppm ZnO, 420 ppm of antibiotics (Antibiotic group; **AB),** basal diet supplemented with 2x10⁹ CFU/kg *Saccharomyces cerevisiae var. boulardii* CNCM I-1079 **(SB).** Fecal samples were collected from 13 and 22 sows per treatment, respectively for primiparous and parity 2 sows. The same sows were sampled 2 days after move to farrowing house, and one day after farrowing. Fecal samples were collected from 10 piglets per treatment group, so 60 piglets in total. Piglets were sampled on weaning day, at day 6 and day 20 post-weaning.

### Microbial DNA extraction and 16S rRNA gene sequencing

Fecal samples collected were processed as followed: microbial DNA was extracted from 40-60 mg of feces using ZR-96 Soil Microbe DNA Kit^{™} (Zymo Research, Freiburg, Germany) according to the manufacturer's instruction. A 15 min bead beating step at 30 Hz was applied using a Retsch MM400 Mixer Mill. The V4 and V5 hypervariable regions of the 16S rRNA gene were amplified using the following primers:

| **V4-V5 region** | |
|---|---|
| V4V5-515F | 5'- CTTTCCCTACACGACGCTCTTCCGATCTGTG**Y**CAGC**M**GCCGCGGTA -3' |
| V4V5-928R | 5'- GGAGTTCAGACGTGTGCTCTTCCGATCTCCCCG**Y**CAATTC**M**TTTRAGT -3' |

High-throughput sequencing was performed on a MiSeq sequencer using the Reagent Kit v3, according to the manufacturer's instruction (Illumina Inc., San Diego, CA).

### Bioinformatics analyses

Bioinformatics analyses were performed using GenoToul bioinformatics facility (Toulouse, France). Generated paired-end 250 bp sequences were assembled using Flash software (10bp minimum overlap, 10% maximum mismatch). Assembled sequences were processed using FROGS pipeline (Escudié et al., 2018). Briefly, sequences were clustered in Operational Taxonomic Units (OTUs) using SWARM algorithm (Mahé et al., 2014). Chimeric sequences were then detected by samples using UCHIME algorithm and removed from all samples (Edgar et al., 2011). A rarefaction step was applied to each sequencing dataset to avoid bias due to differences in sequencing depth. A filtering step was applied to remove all singletons (i.e. OTU represented by only one read). The generated OTU count table was normalized by total sum scaling. Taxonomic annotation of the OTUs was performed using the SILVA SSU database and BLAST+ and RDP algorithms. BLAST hits with identity and coverage alignments higher than 99% were kept for annotation. Otherwise, species were annotated as unknown and RDP classifier results were used for higher rank. Bootstrap thresholds were set to 0,9 and 0,8 respectively for annotation at the genus rank and higher ranks. Phylum, family and genus relative abundance tables were generated.

### Statistical analyses

*Zootechnical performances.* Average daily gain (ADG), feed intake (FI), and feed conversion ratio (FCR) were performed by a MIXED procedure of SAS (The SAS Stat. v.9.3) for repeated measurements. The statistical model accounted for the main effects of treatment of the sow, treatment of the piglet, and time, also considering the interactions. Significance level was fixed for A,B P≤0.01 and a,b P≤0.05, while 0.05<P≤0.1 was considered as a trend.

*Microbiota statistical analyses.* They were carried out using R software and RStudio software. A Kruskal-Wallis Rank Sum Test followed by Pairwise Test for Multiple Comparisons of Mean Rank Sums (Conover-Test, PMCMR R package) were used to analyze compositional data. Differential analyses were performed only on taxa represented by more than 0,005% of the total sequences. The Benjamini-Hochberg procedure was used to adjust generated P values. Contingency data were analyzed using a Fisher's exact test.

### Results

*Zootechnical performance.* A supplementation of sows with *Saccharomyces cerevisiae var. boulardii* CNCM I-1079 resulted in a significant increase in body weight of the piglets at weaning (Tableau 1). Furthermore, an effect of the supplementation of the sows is showed on growth performance on post-weaning performance, up to 35 days of age (Table 2).

These results demonstrates that when the sows were fed with *Saccharomyces cerevisiae var. boulardii* CNCM I-1079, the growth of their piglets was stronger than the growth obtained in the piglets which mothers were not fed with *Saccharomyces cerevisiae var. boulardii* CNCM I-1079.

**Table 1- Effect of a supplementation with Saccharomyces cerevisiae var. boulardii CNCM I-1079 (LSB) of the sow diet on performance at weaning. ADFI: Average Daily Feed Intake**

| | control | LSB | Standard Error | P value |
|---|---|---|---|---|
| ADFI (kg) | 4.2 | 4.3 | 0.1 | 0.63 |
| Average weaning weight (kg) | 5.9 | 6.3 | 0.1 | 0.03 |
| Mortality (%) (LS means) | 2.8 | 1.9 | 0.01 | 0.22 |

**Table 2 - Effect of a supplementation with Saccharomyces cerevisiae var. boulardii CNCM I-1079 (LSB) of the sow and the post-weaning diets on growth performance of piglets; ADG: Average Daily Gain; ADFI: Average Daily Feed Intake; FCR: Feed Conversion ratio.**

| *Dependent Variable* | *LSB_SOW* | *Mean* | *Std. Error* | |
|---|---|---|---|---|
| *ADG 0-35* | *NO* | *311,346* | *9,426* | ***0.014*** |
| | *YES* | *346,488* | *9,426* | |
| *ADFI* | *NO* | *416,791* | *11,796* | ***0.017*** |
| | *YES* | *458,931* | *11,796* | |
| *FCR* | *NO* | *1,343* | *0,027* | *0.741* |
| | *YES* | *1,33* | *0,027* | |

*Microbiota analysis.* In sows, we evidenced an effect of *Saccharomyces cerevisiae var. boulardii* CNCM I-1079 supplementation on the microbiota alpha-diversity. A higher homogeneity of the samples was observed within *Saccharomyces cerevisiae var. boulardii* CNCM I-1079 group.

*Saccharomyces cerevisiae var. boulardii* CNCM I-1079 supplementation in sows was associated with a significant higher relative abundance of the phylum of *Fibrobacteres* before farrowing (Figure 4).

In addition, we observed a higher frequency of *Fibrobacter intestinalis* in piglets whose mothers were fed with *Saccharomyces cerevisiae var. boulardii* CNCM I-1079 supplementation (Figure 5). Strikingly, those mother effects were persistent up to 20 days post weaning whatever the post-weaning diets. Furthermore, medication in piglets decreased *Fibrobacter intestinalis* frequency; while, interestingly, *Saccharomyces cerevisiae var. boulardii* CNCM I-1079 supplementation in sows delayed the effect of medication in piglets (Figure 5). Such results suggest a maternal imprinting effect when sows are fed with *Saccharomyces cerevisiae var. boulardii* CNCM I-1079, probably by impacting early microbiota colonization of their piglets.

A positive correlation between Fibrobacteres relative abundance in sows before farrowing and piglets performance (average weaning weight) was also seen, as shown in Figure 6.

All these results taken together suggest that the supplementation with *Saccharomyces cerevisiae var. boulardii* CNCM I-1079 led to an increase in the population of *Fibrobacteres* in the colon of the sows, but also in the colon of the piglets which sows were fed with *Saccharomyces cerevisiae var. boulardii* CNCM I-1079. In the first example, in addition to the effect on *Fibrobacteres,* we observed an effect on *Actinobacteria* phylum, and especially on *Collinsella* and *Olsenella,* 2 genera described to be able to metabolize primary bile acids. In the 2 provided examples, the piglets, at the same time, exhibited greater growth.

## Claims

1. A non-therapeutic use of a composition for maintaining or growing the population of microorganisms in the gut of offspring of a monogastric animal,
wherein the monogastric animal is a sow,
wherein the population of microorganisms is from the *Fibrobacteres* phylum,
wherein the composition comprises at least one biologically pure culture of *Saccharomyces cerevisiae var boulardii* strain deposited under accession number I-1079 at the CNCM in an amount effective for maintaining or growing the population of microorganisms in the gut of the offspring, and a suitable carrier, and wherein the composition does not comprise any *Enterococcus* strain, and
wherein the use comprises feeding the composition to the sow during gestation and/or lactation.

2. The use of claim 1, wherein the population of microorganisms in the gut of the offspring of the sow fed with the composition is enhanced by at least 2, 3, 4, 5, 10, 15, 20, 25, 30, 35, 40, 45, 50, 55, 60, 65, 70, 75, 80, 85, 90, 95 or 100% when compared to the microorganism population of offspring of a sow not fed with the composition.

3. A non-therapeutic method of maintaining or growing a population of microorganisms in the gut of offspring of a monogastric animal, the method comprising feeding to the monogastric animal during gestation and/or lactation an effective amount of a composition, wherein the monogastric animal is a sow,
wherein the population of microorganisms is from the *Fibrobacteres* phylum,
wherein the composition comprises at least one biologically pure culture of *Saccharomyces cerevisiae var boulardii* strain deposited under accession number I-1079 at the CNCM and a suitable carrier, and wherein the composition does not comprise any *Enterococcus* strain.

4. The method of claim 3, wherein;
(a) the growth of the population in the gut of the offspring of the sow is enhanced by at least 2, 3, 4, 5, 10, 15, 20, 25, 30, 35, 40, 45, 50, 55, 60, 65, 70, 75, 80, 85, 90, 95 or 100% when compared to the microorganism population of offspring of a sow not fed with the composition;
(b) the method comprises admixing the composition with the basal diet of the animal;
(c) the step of feeding to the sow an effective amount of the composition is done on a daily basis for a period of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 days; and/or
(d) the composition fed to the sow further comprises at least one antibiotic, Zinc Oxide or a mixture thereof, optionally wherein the antibiotic is Amoxicillin, Doxycycline, Lyncomycin, colistin, Tiamulin, or any combinations thereof.

5. The use of claim 1 or 2 or the method of claim 3 or 4, wherein the microorganisms are from the *Fibrobacteres* genus, optionally wherein the microorganisms are a *Fibrobacter intestinalis* strain.

6. The use of any one of claims 1, 2 or 5 or the method of any one of claims 3 to 5, wherein the population of microorganisms enhances average daily gain of the offspring of the sow.

7. The use of any one of claims 1, 2, 5 or 6 or the method of any one of claims 3 to 6, wherein:
(a) the suitable carrier is feed;
(b) the at least one biologically pure culture of *Saccharomyces cerevisiae var boulardii* strain is in an amount of at least 1x10⁹ CFU per kilogram of the composition; and/or
(c) the composition is in the form of a gelatin capsule, a pressed tablet, a gel cap, an animal feed, or a liquid beverage.

8. The use of any one of claims 1, 2 or 5 to 7 or the method of any one of claims 3 to 7, wherein the composition further comprises at least one additional microorganism strain.

9. The use of any one of claims 1, 2 or 5 to 8 or the method of any one of claims 3 to 8, wherein the composition does not comprise any bacterial strain and/or any additional yeast strain, or does not comprise any additional microorganism strain.

## Patentansprüche

1. Nicht-therapeutische Verwendung einer Zusammensetzung zur Erhaltung oder Vermehrung der Population von Mikroorganismen im Darm von Nachkommen eines monogastrischen Tiers,
wobei das monogastrische Tier eine Sau ist,
wobei die Population von Mikroorganismen dem Phylum *Fibrobacteres* entstammt,
wobei die Zusammensetzung mindestens eine biologisch reine Kultur von dem Stamm *Saccharomyces cerevisiae var. boulardii,* hinterlegt unter der Zugangsnummer 1-1079 bei der CNCM, in einer Menge umfasst, die zur Erhaltung oder Vermehrung der Population von Mikroorganismen im Darm der Nachkommen wirksam ist, und einen geeigneten Träger, und wobei die Zusammensetzung keinen *Enterococcus-Stamm* umfasst, und
wobei die Verwendung das Verfüttern der Zusammensetzung an die Sau während der Trächtigkeit und/oder Laktation umfasst.

2. Verwendung nach Anspruch 1, wobei die Population von Mikroorganismen im Darm der Nachkommen der mit der Zusammensetzung gefütterten Sau um mindestens 2, 3, 4, 5, 10, 15, 20, 25, 30, 35, 40, 45, 50, 55, 60, 65, 70, 75, 80, 85, 90, 95 oder 100 % erhöht ist, verglichen mit der Mikroorganismenpopulation der Nachkommen einer Sau, die nicht mit der Zusammensetzung gefüttert wurde.

3. Nicht-therapeutisches Verfahren zur Erhaltung oder Vermehrung einer Population von Mikroorganismen im Darm von Nachkommen eines monogastrischen Tiers, wobei das Verfahren das Füttern des monogastrischen Tiers mit einer wirksamen Menge einer Zusammensetzung während der Trächtigkeit und/oder Laktation umfasst, wobei das monogastrische Tier eine Sau ist,
wobei die Population von Mikroorganismen dem Phylum *Fibrobacteres* entstammt,
wobei die Zusammensetzung mindestens eine biologisch reine Kultur von dem Stamm *Saccharomyces cerevisiae var. boulardii,* hinterlegt unter der Zugangsnummer 1-1079 bei der CNCM, und einen geeigneten Träger umfasst, und wobei die Zusammensetzung keinen *Enterococcus*-Stamm umfasst.

4. Verfahren nach Anspruch 3, wobei
a) die Vermehrung der Population im Darm der Nachkommen der Sau um mindestens 2, 3, 4, 5, 10, 15, 20, 25, 30, 35, 40, 45, 50, 55, 60, 65, 70, 75, 80, 85, 90, 95 oder 100 % erhöht ist, verglichen mit der Mikroorganismenpopulation der Nachkommen einer Sau, die nicht mit der Zusammensetzung gefüttert wurde.
b) das Verfahren umfasst das Beimischen der Zusammensetzung zum Grundfutter des Tiers umfasst;
c) der Schritt des Fütterns der Sau mit einer wirksamen Menge der Zusammensetzung täglich über einen Zeitraum von mindestens 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 oder 60 Tagen erfolgt; und/oder
d) die Zusammensetzung, mit der die Sau gefüttert wird, ferner mindestens ein Antibiotikum, Zinkoxid oder eine Mischung davon umfasst, wobei das Antibiotikum optional Amoxicillin, Doxycyclin, Lincomycin, Colistin, Tiamulin oder beliebige Kombinationen davon ist.

5. Verwendung nach Anspruch 1 oder 2 oder Verfahren nach Anspruch 3 oder 4, wobei die Mikroorganismen der Gattung *Fibrobacteres* entstammen, wobei die Mikroorganismen optional ein *Fibrobacter intestinalis*-Stamm sind.

6. Verwendung nach einem der Ansprüche 1, 2 oder 5 oder Verfahrens nach einem der Ansprüche 3 bis 5, wobei die Population von Mikroorganismen die durchschnittliche tägliche Zunahme der Nachkommen der Sau verbessert.

7. Verwendung nach einem der Ansprüche 1, 2, 5 oder 6 oder Verfahren nach einem der Ansprüche 3 bis 6, wobei:
a) der geeignete Träger Futtermittel ist;
b) die mindestens eine biologisch reine Kultur des Stammes *Saccharomyces cerevisiae var. boulardii* in einer Menge von mindestens 1 × 10⁹ KBE pro Kilogramm der Zusammensetzung vorliegt; und/oder
c) die Zusammensetzung in Form einer Gelatinekapsel, einer gepressten Tablette, einer Gelkapsel, eines Tierfutters oder eines flüssigen Getränks vorliegt.

8. Verwendung nach einem der Ansprüche 1, 2 oder 5 bis 7 oder Verfahren nach einem der Ansprüche 3 bis 7, wobei die Zusammensetzung ferner mindestens einen zusätzlichen Mikroorganismenstamm umfasst.

9. Verwendung nach einem der Ansprüche 1, 2 oder 5 bis 8 oder Verfahren nach einem der Ansprüche 3 bis 8, wobei die Zusammensetzung keinen Bakterienstamm und/oder keinen zusätzlichen Hefestamm oder keinen zusätzlichen Mikroorganismenstamm umfasst.

## Revendications

1. Utilisation non thérapeutique d'une composition pour maintenir ou accroître la population de micro-organismes intestinaux de la progéniture d'un animal monogastrique ;
l'animal monogastrique étant une truie,
la population de micro-organismes appartenant au phylum des *Fibrobacteres,*
la composition comprenant au moins une culture biologiquement pure de la souche *Saccharomyces cerevisiae var. boulardii,* enregistrée sous le numéro d'ordre I-1079 auprès de la CNCM, en une quantité efficace pour maintenir ou accroître la population de micro-organismes intestinaux de la progéniture, ainsi qu'un vecteur approprié, et la composition ne comprenant aucune souche *d'Enterococcus,* et
l'utilisation comprenant l'administration par voie alimentaire de la composition à la truie pendant la gestation et/ou la lactation.

2. Utilisation selon la revendication 1, dans laquelle la population de micro-organismes intestinaux de la progéniture de la truie nourrie avec la composition est augmentée d'au moins 2, 3, 4, 5, 10, 15, 20, 25, 30, 35, 40, 45, 50, 55, 60, 65, 70, 75, 80, 85, 90, 95 ou 100 % en comparaison de la population de micro-organismes de la progéniture d'une truie non nourrie avec la composition.

3. Méthode non thérapeutique destinée à maintenir ou à accroître la population de micro-organismes intestinaux de la progéniture d'un animal monogastrique, le procédé comprenant l'administration à l'animal monogastrique, par voie alimentaire, pendant la gestation et/ou la lactation, d'une quantité efficace d'une composition ; l'animal monogastrique étant une truie,
la population de micro-organismes appartenant au phylum des *Fibrobacteres,*
la composition comprenant au moins une culture biologiquement pure de la souche *Saccharomyces cerevisiae var. boulardii,* enregistrée sous le numéro d'ordre I-1079 auprès de la CNCM, et un vecteur approprié, et la composition ne comprenant aucune souche *d'Enterococcus.*

4. Méthode selon la revendication 3, dans laquelle :
(a) la croissance de la population de micro-organismes intestinaux de la progéniture de la truie est augmentée d'au moins 2, 3, 4, 5, 10, 15, 20, 25, 30, 35, 40, 45, 50, 55, 60, 65, 70, 75, 80, 85, 90, 95 ou 100 % en comparaison de la population de micro-organismes de la progéniture d'une truie non nourrie avec la composition,
(b) la méthode comprend le mélange de la composition avec l'alimentation de base de l'animal,
(c) l'étape consistant à administrer à la truie, par voie alimentaire, une quantité efficace de la composition est réalisée quotidiennement pendant une période d'au moins 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 ou 60 jours, et/ou
(d) la composition administrée à la truie par voie alimentaire comprend en outre au moins un antibiotique, de l'oxyde de zinc ou un mélange de ceux-ci, l'antibiotique pouvant éventuellement être l'amoxicilline, la doxycycline, la lincomycine, la colistine, la tiamuline ou toute combinaison de celles-ci.

5. Utilisation selon la revendication 1 ou 2 ou méthode selon la revendication 3 ou 4, dans laquelle les micro-organismes appartiennent au genre *Fibrobacteres,* les micro-organismes pouvant éventuellement être une souche de *Fibrobacter intestinalis.*

6. Utilisation selon l'une quelconque des revendications 1, 2 ou 5 ou méthode selon l'une quelconque des revendications 3 à 5, dans laquelle la population de micro-organismes augmente le gain moyen quotidien de la progéniture de la truie.

7. Utilisation selon l'une quelconque des revendications 1, 2, 5 ou 6, ou méthode selon l'une quelconque des revendications 3 à 6, dans laquelle :
(a) le vecteur approprié est un aliment pour animaux,
(b) l'au moins une culture biologiquement pure de la souche *Saccharomyces cerevisiae var. boulardii* est présente en une quantité d'au moins 1 × 10⁹ UFC par kilogramme de composition, et/ou
(c) la composition se présente sous la forme d'une capsule de gélatine, d'un comprimé, d'une gélule, d'un aliment pour animaux ou d'une boisson liquide.

8. Utilisation selon l'une quelconque des revendications 1, 2 ou 5 à 7, ou méthode selon l'une quelconque des revendications 3 à 7, dans laquelle la composition comprend en outre au moins une souche de micro-organisme supplémentaire.

9. Utilisation selon l'une quelconque des revendications 1, 2 ou 5 à 8, ou méthode selon l'une quelconque des revendications 3 à 8, dans laquelle la composition ne comprend aucune souche bactérienne et/ou aucune souche de levure supplémentaire, ou ne comprend aucune souche de micro-organisme supplémentaire.
